Europäisches Patentamt

European Patent Office

Office européen des brevets

(11) Numéro de publication: **0 468 122 A1**

## DEMANDE DE BREVET EUROPEEN

(21) Numéro de dépôt: **90440069.4**

(22) Date de dépôt: **26.07.90**

(51) Int. Cl.5: **A61K 31/785**, A61K 31/155

(43) Date de publication de la demande:
**29.01.92 Bulletin 92/05**

(84) Etats contractants désignés:
**AT BE CH DE DK ES FR GB GR IT LI NL SE**

(71) Demandeur: **Andermann, Guy**
**2, Rond-Point Esplanade**
**F-67000 Strasbourg(FR)**

(72) Inventeur: **Andermann, Guy**
**2, Rond-Point Esplanade**
**F-67000 Strasbourg(FR)**

(54) **Composition thérapeutique à base de polihexanide pour le traitement des manifestations cutanées, oculaires et génitales de l'herpès.**

(57) La présente invention concerne des compositions thérapeutique à base de polihexanide pour le traitement de diverses formes d'herpés (cutané, oculaire et génital).

Compositions thérapeutiques, caractérisées en ce qu'un principe actif, non toxique le polihexanide, est dissout ou dispersé dans un véhicule adéquat pour permettre une application convenable sur la peau et les muqueuses, tel qu'une efficacité thérapeutique peut être obtenue après quelques jours de traitement topique.

EP 0 468 122 A1

La présente invention concerne un médicament nouveau, utile plus particulièrement dans le traitement topique des affections cutanées et oculaires à virus, notamment l'herpès cutané labial, génital et ophtalmique.

Le traitement de l'herpès pose un véritable problème thérapeutique, dans la mesure où peu de produits sont actifs lorsqu'ils sont administrés par voie topique. On a récemment décrit des activités intéressantes à propos de l'acyclovir, ou de l'isoprinosine. Mais ces produits doivent obligatoirement être administrés par voie systémique pour être actifs.

Cette voie d'administration n'est pas sans effets secondaires, et son prix de revient est très élevé.

L'utilisation topique de ces mêmes molécules s'est montrée plus décevante dans la mesure où il est nécessaire d'administrer des compositions pharmaceutiques ayant une pénétration optimale au niveau du site d'action. Les produits actuellement commercialisés sous forme de pommade ou de gel, à base d'idoxuridine, de cytarabine, d'iododésoxycytidine ou même d'acyclovir sont d'une activité thérapeutique décevante quand ils sont administrés localement. Cette faible activité thérapeutique s'exprime quelle que soit la forme pharmaceutique, que ce soit sous forme de collyre dans les affections ophtalmiques, ou sous forme de pommade, lotion ou gel dermique dans les affections cutanées ou génitales.

Des tentatives d'utiliser diverses formes d'interferon se sont également avérées décevantes du point de vue thérapeutique, en particulier dans la kératite herpétique.

Les recherches effectuées par la demanderesse, in vitro et in vivo, ont permis de découvrir un moyen thérapeutique permettant d'éviter les inconvénients mentionnés plus haut.

La présente invention a pour objet un médicament topique, utilisé notamment pour le traitement local des maladies virales à virus à enveloppe lipidique du type herpès ou autre. Il contient une quantité pharmaceutiquement acceptable et active de polihexanide, ou polyhexaméthylène biguanide.

Un médicament topique suivant l'invention peut être sous forme de gel dermique ou oculaire, de créme dermique ou de pommade, de pommade ophtalmique, de lotion ou même de formes uni-doses comme les capsules de gélatine stériles, ou les flacons uni-doses de 0,5 à 2 ml en plastique polyéthylène ou polypropylène.

Le polihexanide est un dérivé polymère de biguanide, utilisé auparavant dans l'industrie alimentaire sous le nom commercial de Vantocil. Une autre application décrite dans le brevet Fr 81 22541, consiste à utiliser la substance comme désinfectant de lentilles de contact. Des dérivés du produit ont été commercialisés récemment comme conservateurs dans des solutions de lentilles de contact. Il peut être considéré comme un produit particulièrement sûr en ce qui concerne sa biocompatibilité avec les tissus humains, en particulier la peau et les muqueuses oculaires. Il ne provoque de ce fait aucune irritation ou allergie avec la peau et l'oeil humain ou animal.

Le polihexanide se présente en solution aqueuse à 20 p.cent. Ses propriétés à la fois hydrophiles et surtout lipophiles permettent d'une part la préparation de médicaments sous plusieurs formes d'administration d'une part (gels dermiques, collyres aqueux et pommades ophtalmiques par exemple), et d'autre part d'avoir une activité remarquable sur les virus à enveloppe lipidique comme celui du virus de l'herpès, d'autre part.

Les faibles coûts de production de ces médicaments en font des agents thérapeutiques particulièrement intéressants d'un point de vue économique.

En effet, si les produits antiherpétiques actuellement commercialisés ont fait la preuve d'une certaine activité, ce sont des produits qui doivent être délivré sur prescription médicale, et qui sont par ailleurs très onéreux. La stricte surveillance médicale, liée à leur toxicité, est également un handicap majeur. En effet, l'éclosion d'une crise d'herpès est liée au comportement très particulier du virus herpétique dans l'organisme humain. Il est désormais établi et reconnu que le virus sera d'autant plus virulent que l'organisme hôte sera plus faible, en particulier dans les cas d'infections microbiennes, au cours du cycle menstruel, et même lors de stress psychiques importants, qui se caractérisent par un affaissement des défenses de l'organisme. Par ailleurs, l'herpès se caractérise par des récidives nombreuses chez les mêmes individus qui y sont sensibles. L'affection débute toujours par une phase primaire de démangeaison, que le malade herpétique sait souvent reconnaitre aisément.

Or, le succès de toutes les thérapeutiques antiherpétiques dépend étroitement du moment auquel est administré le produit actif. Plus le médicament antiherpétique sera administré tôt, plus il sera efficace. Les médicaments actuellement disponibles sur le marché, sont tous des produits qui doivent être administrés sous stricte surveillance, et nécessitent une ordonnance médicale pour être délivrés. Cette obligation retarde significativement l'administration de ces médicaments, car le malade aura besoin de consulter pour avoir une prescription médicale, avant de pouvoir accéder au traitement.

Lorsque le malade soumis à des récidives fréquentes a laissé passer la phase primaire des signes, il est souvent trop tard pour retirer un bénéfice thérapeutique d'un traitement quel qu'il soit. C'est ainsi que l'apparition de placard et d'éruptions cutanées rouges surélevées est souvent l'indice que le virus a déjà atteint une phase de maturation clinique telle que les virostatiques, comme la vidarabine, l'iododésoxyuridine, ou l'acyclovir ne permettent plus de juguler la crise herpétique. Seuls les virucides, comme le polihexanide, par exemple, peuvent encore être d'une efficacité thérapeutique non négligeable.

Lorsque plus tard encore apparaissent les vésicules cutanées, qui se déssèchent progressivement, la maladie évolue vers la guérison, même sans traitement, parfois accompagnée de douleurs d'intensité variable.

Le médicament qui fait l'objet de la présente invention, permet de pallier à tous ces inconvenients. Il se caractérise par l'avantage de pouvoir être présente sous diverses formes pharmaceutiques topiques, aqueuses ou non, sous forme de solution limpide, d'émulsions, comme les solutions uni ou multi-doses, les collyres, les gels, lotions ou crèmes dermiques, et les pommades ophtalmiques.

Ces avantages de presentations multiples autorisent une administration à la fois dans l'herpès oculaire, l'herpès cutané et l'herpès vaginal. Ces formes pharmaceutiques permettent une fabrication industrielle peu onéreuse, ce qui constitue un autre avantage de l'invention. Cet avantage se cumule avec l'absence de toxicité des produits, autorisant de ce fait, un achat rapide et peu couteux par le malade lui-même.

La rapidité d'administration du produit par le patient qui accède au médicament dès l'apparition des premiers signes, comme indiqué plus plus haut, autorise une rémission ou une guérison des signes cliniques en moins de 5 jours.

En pratique, la composition de l'invention comprend généralement des quantités de polyhexanide comprises entre 0,0002 % et 2 %, exprimées en base pure. Le produit est généralement utilisé sous forme de sel hydrosoluble, comme le chlorhydrate. D'autres sels de polihexanide peuvent également être utilisés, comme par exemple, le gluconate, l'acétate ou le diiséthionate.

Le produit pharmaceutique peut avantageusement être combiné avec des promoteurs d'absorption qui augmenteront encore l'efficacité du produit en permettant une meilleure pénétration à travers la paroi du virus. De préférence, ces adjuvants ne doivent pas présenter d'incompatibilté avec le polihexanide, dont l'efficacité propre risque d'être inhibée par leur présence. Certains ammoniums quaternaires par exemple sont incompatibles avec le principe actif, dans la mesure oû ils forment des complexes inactifs avec le polihexanide dans certaines zones de concentration du polihexanide.

Le pH du produit administré à l'homme doit être également étudié soigneusement. En effet, des essais thérapeutiques ont montré d'une part que des compositions à pH trop bas (4 ou 5) étaient mal tolérées par les malades. La compliance au traitement avait dans ce cas diminué, ce qui est préjudiciable à l'efficacité thérapeutique.

Un pH trop élevé (au dessus de 9) du produit a conduit à une diminution significative de l'efficacité antivirale des produits à base de polihexanide.

De préférence, le pH des médicaments contenant du polihexanide pour le traitement topique des diverses formes d'herpès, doit être compris entre 5,5 et 8. Les formes oculaires (collyre et pommade ophtalmique) doivent être comprises entre 6 et 7,5 en solution tamponnée ou non.

Des essais in vitro et des essais thérapeutiques chez l'homme, à diverses concentrations de polihexanide, ont permis de mettre en évidence l'efficacité du polihexanide dans le traitement de l'herpès cutané, oculaire et génital.

Des posologies diverses ont été comparées au cours de ces essais. Il a pu être démontré par ces recherches que 3 à 5 applications par 24 heures pendant 3 à 4 jours, ont permis la guérison des signes cliniques de l'herpès, que la localisation soit cutanée ou génitale. Les atteintes oculaires ont nécessité des administrations plus fréquentes, (6 fois par 24 heures pendant 5 jours), pour qu'une amélioration soit visible.

Dans tous les essais cliniques effectués, on n'a pu mettre en évidence d'effets secondaires, ou de résistance au traitement, comme la littérature le rapporte à propos de l'I.D.U., de la vidarabine ou d'autres produits topiques. Même l'acyclovir n'a pas toujours donné des résultats aussi satisfaisants que ceux que les présentes recherches ont permis de démontrer.

Par contre, dans tous les cas, on a pu mettre en évidence l'importance de la vitesse à laquelle le produit était administré après les premiers signes de démangeaison cutanée ou génitale.

L'invention sera mieux comprise par les exemples non limitatifs qui suivent. Ils concernent à la fois des compositions suivant l'invention, et des séries d'éssais cliniques utilisant l'un ou l'autre exemple de l'invention.

Exemple 1 gel dermique pour le traitement de l'herpès cutané comprenant:
- 0,025 g % de polihexanide
- 0,025 g % de saccharinate d'alkylammonium
- 6,00 g % de lauryléther polyoxyéthyléné
- 2 g % de polymère carboxyvinylique

- 2 g % d'oxyde de titane
- 5 g % d'acétone
- une quantité suffisante de triéthanolamine pour un pH de 4,5
- qsp 100 g % d'eau purifiée

La composition thérapeutique selon l'invention a été administrée 4 fois par 24 heures par application biquotidienne, sous forme de massage sur les lésions atteintes d'herpès. Avec cette posologie, l'efficacité thérapeutique a pu être démontrée au bout de 4 jours grâce à des essais cliniques controles en double aveugle par rapport à un placebo. La population de malades inclus dans l'esais était composée uniquement de patients souffrant d'herpès recidivant, et qui connaissaient bien les signes précurseurs d'apparition de l'affection. La composition de l'invention a été administrée au plus tard au stade I de l'éruption, où l'herpès se manifeste par une rougeur oedémateuse avec début de vésiculisation.

Dans la série des malades traités par le polihexanide, 76 % des malades montraient des lésions qui se résorbaient et devenaient inapparentes après 4 jours. Dans le groupe placébo, il a fallu plus de 20 jours pour atteindre un résultat comparable chez 80 % des malades. La tolérance a été excellente chez tous les malades traités avec le principe actif.

Exemple 2 collyre pour le traitement de l'herpés oculaire comprenant:

```
- polihexanide           0,025 g %
- phosphate mopotassique  0,113 g %
- phosphate dipotassique  0,250 g %
- chlorure de magnesium   0,700 g %
- hydroxyethylcellulose   0,500 g %
- eau purifiée        qsp 100 ml
```

20 lapins sont divisés en deux groupes de 10 animaux chacun. La cornée de l'oeil droit de chacun est infectée avec une goutte de solution contenant le virus herpès simplex à dix puissance moins sept/ml, aprés avoir fait 2 scarifications de la cornée de 4 à 5 mm chacune. L'instillation de la suspension virale est suivie d'un massage de la paupière pendant 30 secondes. Le premier groupe (10 lapins) a servi de témoin, le second groupe a reçu 4 instillations d'une goutte de la composition de l'invention à raison d'une instillation toutes les 2 heures pendant cinq jours consécutifs pour une première évaluation.

Au 3ième jour de l'exposition au virus dans le groupe témoin, les lapins ont présenté ds kératites, alors que le groupe traité montrait des inflammations et des secrétions moins importantes.

Au 5ième jour après l'infection, le groupe traité présentait significativement moins de kératite que le groupe témoin non traité, et les prélèvements oculaires dans ce groupe étaient stériles. Les lésions ont guéri définitivement après 4 semaines de traitement avec la composition de l'invention, alors que les lapins du groupe témoin non traités montraient encore une kératite importante après la fin de l'expérience.

**Revendications**

1. Medicament, notamment pour le traitement des diverses formes d'herpès viral, caractérisé en ce qu'il contient une quantité pharmaceutiquement active et biologiquement compatible de polihexanide en association ou non avec unpromoteur d'absorption.

2. Médicament selon la revendication 1, caractérisé en ce qu'il se présente sous la forme d'une solution aqueuse de pH compris entre 5,5 et 8 contenant entre 0,0002 g % et 2 g % en poids de polihexanide.

3. Médicament, suivant l'une des revendications 1 et 2, caractérisé en ce qu'il est préparé et conditionné sous la forme galénique d'un collyre, d'une pommade vaselinée, d'une crème dermique, d'un gel ou d'une lotion.

4. Médicament selon la revendication 3, caractérisé en ce qu'il contient des excipients non toxiques de collyre aqueux, comme des tampons, des isotonisants, des épaississants cellulosiques ou non, en quantités de 0,01 à 1 g% du poids de la composition aqueuse totale.

5. Médicament, suivant la revendication 3, caractérisé en ce qu'il se trouve sous forme d'un gel dermique, renfermant principalement un promoteur d'absorption cutané de type alkylammonium, des dérivés polyoxyethyléniques de l'alcool laurique en tant que tensio-actif non ionique.

6.  Médicament, suivant l'une des revendications 1 à 5, caractérisé en ce qu'il est conditionné soit dans des formes uni-doses en plastique polyéthylène ou polypropylène, soit dans des capsules ou des gélules de gélatine.

7.  Composition thérapeutique selon la revendication 3 et 5, carctérisée en ce que, dans sa présentation, sous forme de gel cutané, elle contient essentiellement du saccharinate d'alkyldiméthylbenzylammonium en tant que promoteur d'absorption, un dérivé polyoxyéthyléné de l'alcool laurique en tant qu'agent tensio-actif, du polymère carboxyvinylique comme épaississant, et un véhicule hydro-acétonique

8.  Médicament, suivant la revendication 3, caractérisé en ce qu'il se trouve sous forme de collyre aqueux, renfermant principalement un tampon phosphate, du chlorure de sodium, du chlorure de magnésium, à un pH compris entre 6 et 7,5.

9.  Utilisation de la composition suivant les revendications 6 à 8 pour la préparation d'un médicament du traitement de l'herpès cutané, oculaire et génital, par application locale sur la peau et les muqueuses.

## DOCUMENTS CONSIDERES COMME PERTINENTS

| Catégorie | Citation du document avec indication, en cas de besoin. des parties pertinentes | Revendication concernée | CLASSEMENT DE LA DEMANDE (Int. Cl.5 ) |
|---|---|---|---|
| X | PATENT ABSTRACTS OF JAPAN vol. 12, no. 462 (C-549)(3309) 05 décembre 88. & JP-A-63 183502 (UENU SEIYAKU) 28 juillet 88. * le document en entier * | 1-9 | A61K31/785 A61K31/155 |
| Y | EP-A-252310 (MERZ & CO.) * revendication 3 *page 7 tab.4:Kombination 0 | 1-9 | |
| Y | GB-A-1434040 (ICI) * revendication 1 * | 1-9 | |
| A | J.APPL.BACTERIOL. vol. 54, 1983, pages 345 - 353; Broxton et al.: "A study of the antibacterial activity of some polyhexamethylene biguanides towards Escherichia Coli ATCC 9738" * page 346, colonne 1, lignes 2 - 10 * * page 348, colonne 1, ligne 18 - colonne 2, ligne 13 * | 1-9 | |
| A | MICROBIOS vol. 40, 1984, Cambridge pages 15 - 22; Broxton et al.: "Binding of some polyhexamethylene biguanides to the cell envelope of Escherichia Coli ATCC 8739" * page 15, lignes 1 - 19 * * page 17, lignes 18 - 23 * | 1-9 | DOMAINES TECHNIQUES RECHERCHES (Int. Cl.5 ) A61K A01N |

Le présent rapport a été établi pour toutes les revendications

| Lieu de la recherche | Date d'achèvement de la recherche | Examinateur |
|---|---|---|
| LA HAYE | 21 SEPTEMBRE 1990 | GERLI P.F.M. |